# EUROPEAN PATENT SPECIFICATION

(11) **EP 2 694 881 B1**
(45) Date of publication and mention of the grant of the patent: **05.08.2015**
(21) Application number: 12711899.0
(22) Date of filing: 04.04.2012
(51) Int. Cl.: A61M 16/16, F24F 3/16, A47C 21/04, A61G 13/10, A61M 16/06, A61M 16/00, A61M 16/10, A61H 35/00, A61H 33/00, A61H 33/06

(54) **TEMPERATURE CONTROLLED LAMINAIR AIR FLOW DEVICE**
TEMPERATURGESTEUERTE LAMINARE LUFTSTROMSVORRICHTUNG
DISPOSITIF DE FLUX D'AIR LAMINAIRE THERMOCOMMANDÉ

(30) Priority: 06.04.2011 US 201161472359 P; 06.04.2011 EP 11002875
(43) Date of publication of application: 12.02.2014
(73) Proprietor: AIRSONETT AB, 262 72 Ängelholm (SE)
(72) Inventor: KRISTENSSON, Dan Allan Robert, S-26263 Ängelholm (SE); SVENSSON, Pål Martin, S-30244 Halmstad (SE)
(74) Representative: Budde Schou A/S
(86) International application number: PCT/EP2012/056222
(87) International publication number: WO 2012/136728

(56) References cited:
- WO-A1-01/94853
- WO-A1-2004/092658
- WO-A1-2007/100286
- WO-A1-2008/136740
- US-A1- 2009 247 065

## Description

### Field of the invention

This invention relates in general to methods for reducing or removing symptoms of atopic dermatitis which utilize Temperature controlled Laminar Air (TLA) flow thereby providing a draught-free clean air personal breathing zone while displacing body convection.

### Background of the invention

### Severe Atopic Dermatitis

Atopic dermatitis (AD) is a chronic inflammatory skin disease which affects 17% of the children and 2% of the adult population and about 2% of the AD patients are classified as suffering from severe AD. Americans are estimated to spend up to $3.8 billion annually on physician services and prescription drugs for the treatment of AD (ref. 1).

Moderate-to-severe AD can have a profound effect on the quality of life for both sufferers and their families. In addition to the effects of intractable itching, skin damage, soreness, sleep loss and the social stigma of a visible skin disease, other factors such as frequent visits to doctors, special clothing and the need to constantly apply messy topical applications all add to the burden of disease. A target population including parts of the moderate population would likely be at least 5 fold greater than the severe population.

Corticosteroids and immune modulators can generally be used to control mild outbreaks. However, severe cases are usually refractory to conventional treatment and require treatment with oral corticosteroids or immunosuppressant such as azathioprine and cyclosporine. The treatment of AD may involve a multimodality approach including airborne protein avoidance, increased frequency of bathing, traditional barrier repair and systemic immunotherapy.

In a recent review from 2010 by Walling and Swick (ref. 8) the following overview of AD treatments was included as table 2:

| **Treatment overview of AD** | |
|---|---|
| Lifestyle interventions | Emollients, bathing technique, |
| | humidification, |
| | avoidance of exacerbants |
| Topical therapy | Topical corticosteroids (first line) |
| | Topical calcineurin inhibitors (second line) |
| | tacrolimus, pimecrolimus |
| | Barrier enhancing creams |
| Phototherapy | Narrowband UVB (311 nm) |
| | Broadband UVB (280-315 nm) |
| | UVA (315-400 nm) |
| | UVA I (340-400 nm) |
| | Psoralen UVA |
| | Extracorporal photochemotherapy |
| Systemic therapies | Traditional Corticosteroids |
| | Cyclosporine |
| | Azathioprine |
| | Methotrexate |
| | Mycophenolate mofetil |
| | Systemic therapies: Biologic Interferon-γ |
| | Immunoglobulin E/Interleukin-5 |
| | Inhibitors |
| | Omalizumab |
| | Mepalizumab |
| | Intravenous immunoglobulin |
| | Tumor necrosis factor-alpha inhibitors |
| | Infliximab |
| | Etanercept |
| | B- and T-cell Inhibitors |
| | Alefacept |
| | Rituximab |
| Ancillary therapies | Antihistamines for control of pruritus |
| | Antibiotics (oral, topical) for control of secondary infection |

D. Wahn *et al.* (ref. 2) evaluated more than 2,000 children 13- to 24 months old with AD and found that severe cases of AD disease was often associated with an increased frequency of sensitization to airborne allergens (20.5% of patients with mild disease had positive testing compared with 45.4% of patients with severe disease). Three studies including a total of more than 4,000 patients demonstrated increased sensitization to airborne proteins including House Dust Mite (HDM) and cat dander in patients with more severe AD (refs. 2, 3, 4 and 5). In this perspective about 0.15% of the US children population suffer from a severe AD associated to airborne allergens. Patients with severe AD often have a comorbid airway disease such as asthma (36%) and/or atopic rhinitis (62%), however, not necessarily a severe airway disease.

Clean air technology is highly effective at removing airborne particles by passing an ambient air-stream through High Efficiency Particulate Air (HEPA) filters. However, the efficiency of HEPA filtration systems depends on air flow dynamics of the environment in which the device is used. In-mixing of contaminated ambient air with filtered air typically diminishes the ultimate efficiency of HEPA filtration, e.g. in providing a purified personal breathing zone.

Several devices have been reported that provide a purified personal breathing zone.

WO2008/058538, US 6,910,961 and US2008/0308106 describe specialized air supply outlets that can be positioned to provide conditioned air for a personal clean-air environment.

US 6,916,238 describes an enclosed clean air canopy that provides a purified personal breathing zone during sleeping hours.

US 7,037,188 describes a bed ventilation system that provides a purified personal breathing zone during sleeping hours.

These devices utilize impulse or forced-blown air to induce and maintain a stream of filtered air, enveloping a point of care. These methods and devices are, however, associated with uncomfortable air flow drafts, dehydration and an overall poor control of the filtered air-stream velocity. Bare parts of the human body are extremely sensitive for air movements during low activity or sleep, thus avoiding drafts and dehydration is pivotal for the long term compliance by patients.

Furthermore, the greater the velocity of the descending laminar air-stream, the more difficult it is to control and direct it to the point of care without in-mixing of ambient air. Hence, high velocities of forced-blown air inevitably invoke turbulent in-mixing of contaminated ambient air in the absence of a canopy or enclosure.

In a TLA device, the velocity of the descending air-stream is determined by the air-temperature difference (i.e. density differences) between the colder, filtered supply air and the ambient air at the level of the point of care. Only minimal impulse is imparted to the air-stream, sufficient to overcome resistance at the outlet nozzle.

Commercially available laminar air flow devices include the Airex M100™ and the EIU™. TLA devices include the Airsonett PROTEXO™.

US 6,702,662 describes a device that utilizes TLA to provide a personal breathing zone. In this device, filtered air is divided into two partial air-streams one of which is cooled, the other heated.

US 2009/0247065 describes devices and methods for improvement of microvascular function using a TLA device delivering a laminar air flow wherein the temperature difference between the filtered air and the ambient air is 0.3 to 3 °C. However, this usage relates to an entirely different field of treatment, i.e. microvascular function and the device, furthermore, operates using a temperature difference of a much wider interval than that disclosed in the present invention (cf. paragraph 0016 of US 2009/0247065).

The use of a TLA device in the treatment of asthma and allergy is disclosed in a marketing brochure published by the company Airsonett AB. The brochure reproduces data from two case reports on individual asthma patients and a pilot study comprising 7 allergic adults.

Sanda *et al.* describes how AD patients were treated in clean rooms. In this study 30 AD patients sensitized to house dust mite (specific IgE RAST score of 3 or higher) but not to pet dander, mold or pollen, were evaluated in the efficacy of clean room treatment in an inpatient hospital for the treatment of AD. Results indicated that 3-4 weeks of 24 hour/day clean room treatment can reduce itchiness and alleviate eczema eruptions (ref. 6).

However, it follows from a more recent review by Hostetler *et al.* (ref. 7) that a substantial number of studies have found no difference in AD frequency or severity with airborne allergen avoidance measures. In one study AD was actually more likely in the HDM avoidance group after one year.

### Brief description of the figures

Figure 1 illustrates convection currents generated by a warm body in a sleeping position.
Figure 2 illustrates a controlled personal breathing zone generated by TLA.
Figure 3 illustrates an embodiment of a device according to the invention.
Figure 4 illustrates embodiments of filtered air-stream temperature adjustment units
Figure 5 illustrates alternative systems for dissipation of excess heat from the air-stream temperature adjustment unit.
Figure 6 illustrates functioning of one embodiment of a nozzle.
Figure 7 illustrates some alternative arrangements of preferred embodiments used in providing a controlled personal breathing zone useful for the treatment of asthma
Figures 8-10 show the reduction of AD symptoms in a 15 year old male patient upon TLA treatment.

### Summary of the invention

The current invention was made in view of the prior art described above and the object of the present invention is to provide a practical and effective method for treating AD.

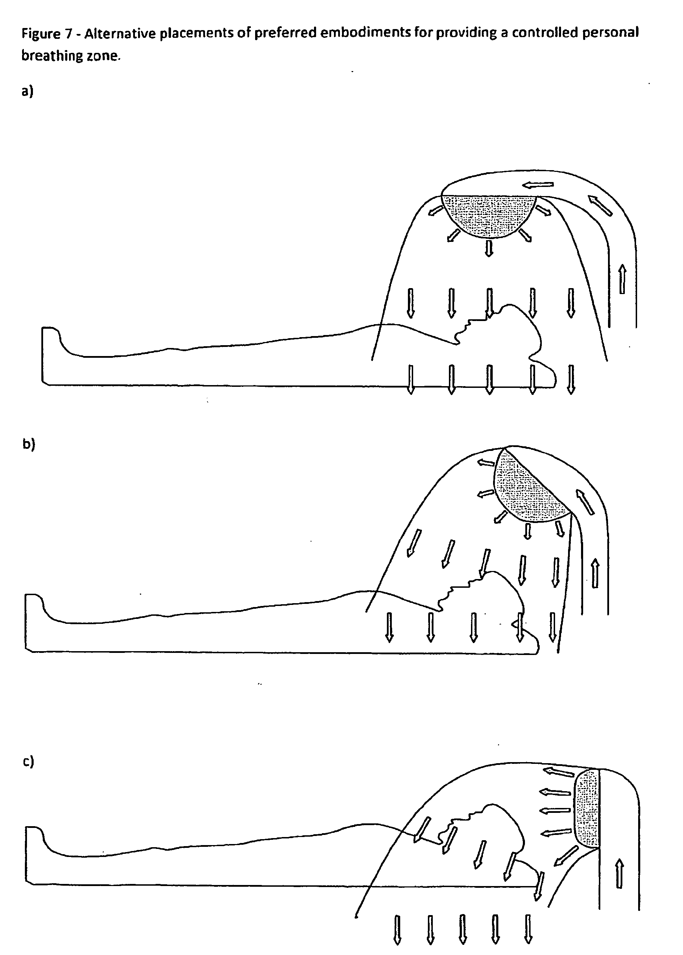

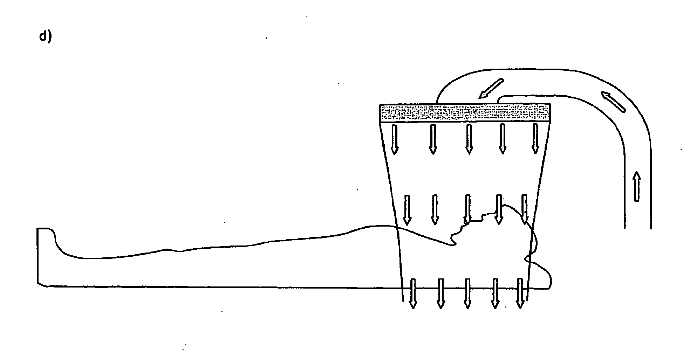

As a solution to this problem it has surprisingly been found that temperature controlled laminar air flow (TLA) can be used to supply a substantially laminar air flow directed to the personal breathing zone (cf. Figure 7) of a patient suffering from AD during situations of or corresponding to sleep whereby allergen avoidance is achieved during at least part of the sleep period whereby symptoms of AD are reduced or removed without the having to place the patient in a HEPA filtered clean room facility.

The TLA treatment is designed to significantly reduce the allergen load in the patient's breathing zone by vertically displacing the contaminations, originating from the bed and the room environment, with a laminar, allergen free, air flow during sleep. The air flow is filtered, slightly cooled and showered over the patient. Due to the higher density, the cooled air descends slowly down, and displaces the contaminants from the breathing zone. In a controlled clinical trial it has surprisingly been found that air 0.5 to < 1 °C cooler than the ambient air effectively displaces warm body convection currents without causing an unpleasant draft whereby symptoms of AD are reduced.

Case studies of patients with moderate to severe (difficult to treat) atopic dermatitis have shown significant effects from nocturnal TLA treatment.

### Detailed description of the invention

The relative particle and allergen concentration in air during situations of or corresponding to sleep is generally higher than in other situations and elsewhere in normal bed- or living-rooms due to body convection. Human body generated convection currents passing the breathing zone carry emissions from the all important reservoirs in the beddings distorted due to movements in the bed.

One way of avoiding particles and allergens is to place the patient 24 hours per day in an environment substantially free of allergens and other airborne particles, i.e. a clean room. Clearly, this is not a feasible approach to treat a large number of patients nor is it convenient for the patient(s).

Devices for providing a controlled breathing zone in the patient's home are known in the art, but none of these have been described to effectively reduce or remove symptoms of atopic dermatitis. The lack of efficacy of certain of these devices is at least in part due to air being blown into the clean air zone resulting in in-mixing of contaminated air and the whirling up of particles present in or on the surface on which the patient is placed, e.g. bedding.

### The principle of TLA

Turbulent in-mixing of ambient air can be avoided by utilizing gravity to induce a laminar air flow, rather than impulse or blowing force. The principle of TLA is that a laminar flow is induced by an air-temperature difference between supply air and ambient air at the point of care. A substantially laminar flow of filtered, colder air, having a higher density than ambient air descends slowly, enveloping the breathing zone of a sleeping person. The TLA principle makes it possible to control the air flow velocity as measured at the point of care. Parts of or the whole temperature control device may be situated before or after the blower device supplying the laminar air flow.

TLA is based upon boundary control and unidirectional orientation of a laminar air supply structure. Stable flow conditions are maintained by introducing a temperature gradient (negative buoyancy) between the cooled supply air and ambient air in the human breathing zone. Entrainment including turbulent diffusion of ambient air into the laminar supply stream is here limited to a minimum. The filtrated and cooled laminar air, with higher density than ambient air, descends slowly enveloping the breathing zone of a person in bed. This downward directed displacement flow will pass physical obstacles in the air-flow path without a substantial effect on the air flow. A free and isothermal jet flow loses momentum after bouncing off physical obstacles. In contrast, the cooled TLA air retains its lower temperature despite interactions with physical obstacles. TLA thus provides improved removal of contaminants from the breathing zone to the floor level.

It has previously been demonstrated that allergens and other airborne particles accumulate in bedding. In the present invention it has been found that a warm human body lying in a bed causes a convection flow transporting a high concentration of allergens and airborne particles to the person's breathing zone. This phenomenon appears independently of any decrease in particle concentration in the ambient air due to operating room air cleaner. Room air cleaner systems thus cannot typically displace body convection and provide a controlled personal breathing zone.

To be effective in providing a controlled personal breathing zone, a TLA device will ideally provide a substantially laminar descending air flow having sufficient velocity to displace convection currents caused by body heat. A warm human body causes a convection air flow having an ascending velocity of over 0.1 m/s and having an air-temperature increased as much as 2 °C above ambient air at body level. An effective TLA device thus typically provide a descending, substantially laminar flow of filtered air with a velocity higher than 0.1 m/s, and in any case, sufficient to break body convection currents of a sleeping (or similar state) person laying in bed.

The present invention provides an easy-to-use non-invasive method for reducing or removing symptoms of atopic dermatitis based on TLA treatment which is suitable for home use without need for isolating the patient.

The word patient in this context is taken to mean any person diagnosed with atopic dermatitis including periodically non-symptomatic persons.

In one embodiment, the invention provides a method for reducing or removing the symptoms of atopic dermatitis in a patient in need thereof by reducing exposure to allergens and other airborne fine particles as measured at the point of care by delivering treated air into the area of a patient's personal breathing zone wherein said treated air descends in a laminar fashion thereby substantially preventing in-mixing of ambient air wherein the air-temperature of the air delivered into the treated air zone - when measured at the point of care - is 0.5 to < 1 °C cooler than the ambient air (such as 0.5 to 0.9 °C or preferably 0.6 to 0.8 °C cooler) surrounding the treated air zone whereby warm body convection currents are displaced without the patient being exposed to unpleasant draft.

In this fashion both inhalation and cutaneous exposure to aeroallergens is avoided in the facial region.

In a preferred embodiment the air is treated by filtration.

Thus, effectively reducing aeroallergen such as pet dander (predominately < 5 µm) and house dust mites (>10 µm) exposure at the point of care.

In a preferred embodiment the temperature difference between the air that is delivered and the ambient air is maintained substantially constant.

### The Device

A suitable device comprises at least one of each of the following: (1) an air inlet, (2) a filter, (3) a blower, (4) an air-temperature adjustment system, (5) an air-temperature control system, (6) an air supply nozzle, and (7) a housing

The one or more air inlets (1) are preferably placed near the floor level of the premises in which the device is utilized, where the layer of coolest air is situated. Alternatively air inlets may be placed higher up in the room, although this typically results in higher energy consumption in that warmer layers of air must be cooled. Preferably, the air inlets are configured in such manner as to keep emission of sound waves during operation to the lowest practicable levels. The more openings included in the device housing, the greater will be the noise levels perceived by the patient. In some embodiments, the air inlets may be associated with a pre-filter that also serves as a sound damper. In other embodiments, a HEPA filter that provides ultimate filtration of the supply air may be situated directly at the air inlets.

The filter (2) is preferably a high efficiency particulate air filter, preferably HEPA class H11, or higher if needed at point of care. In other embodiments, any suitable filter media or device adapted to filter particles or gases unwanted at the point of care may be used. Including for example any combinations of fiberglass and/or polymer fiber filters, or electro static filters, or hybrid filters (i.e. charging incoming particles and/or the filter media), or radiation methods (i.e. UV-light), or chemical and/or fluid methods, or activated carbon filters or other filter types.

While filter effectiveness is preferably high and stable over time, the resistance to air flow, or "pressure drop" generated by the filter is preferably kept low. Increased pressure drop generated by the filter, the device housing, the air delivery nozzle and other components and air channels of the device calls for increased blower speed which in turn generates unwanted noise. In preferred embodiments, pressure drop of a suitable filter is generally lower than 50 Pa. When using the preferred embodiment of HEPA filter using fiberglass or polymer fiber filter media, pressure drop is generally minimized by maximizing the active filter media area.

In preferred embodiments, HEPA filters are comprised of randomly arranged fibers, preferably fiberglass, having diameters between about 0.5 and 2.0 micron, and typically arranged as a continuous sheet of filtration material wrapped around separator materials so as to form a multi-layered filter. Mechanisms of filtration may include at least interception, where particles following a line of flow in the air-stream come within one radius of a fiber and adhere to it; impaction, where large particles are forced by air-stream contours to embed within fibers; diffusion, where gas molecules are impeded in their path through the filter and thereby increase the probability of particle capture by fibers. In some embodiments, the filter itself may comprise the air supply nozzle through which supply air is delivered.

Alternatively or complementary to a HEPA filter, any suitable air treatment system can be used, including at least a humidifier or a dehumidifier, ionizer, UV-light, or other system that provides air treatment beneficial at the point of care.

Preferred embodiments of a device according to the invention comprise an electronic filter identification system. When a filter becomes clogged with particles, its effective area is decreased and its pressure drop accordingly increased. This results in lower air flow, which reduces overall effectiveness of the device. Accordingly it is preferable that patients change the filter within the recommended service interval. To facilitate proper use, preferred embodiments provide a filter management system that indicates when a filter should be changed. Each filter can be equipped with a unique ID that permits the TLA device to distinguish previously used filters from unused ones. Filter identification systems can be provided RFID, bar codes, direct interconnections, attachments such as iBUTTON™ circuits on a circuit board on the filter. It might also be possible to read or read and store other data than the serial number on the filter by this system. Information about the most appropriate air flow according to the filter type can for instance be supplied with the filter and be read automatically by the system.

The blower (3) generates air flow needed to feed a sufficiently large stream of air and to create pressure sufficient to overcome the pressure drop generated by the device. The blower may be of any suitable design, preferably comprising a fan impeller/blower rotor driven by an electric motor. Preferred embodiments are adapted so as to generate minimal noise during operations.

Blower noise is generally minimized by maximizing the size of the rotating rotor and minimizing the rotation per minute.

In preferred embodiments the fan generates a flow of filtered air through the device is less than 500 m³/h, such as less than 400 m³/h, preferably less than 300 m³/h, such as less than 250 m³/h, more preferably less than 225 m³/h, such as less than 200 m³/h, and even more preferably less than 175 m³/h, such as less than 150 m³/h.

The temperature adjustment system (4) cools and/or warms the supply air. In preferred embodiments, both heating and cooling are provided by a thermoelectric Peltier module. As is known in the art, a Peltier module can provide both heating and cooling depending on the polarity of the applied voltage or the direction of its operating current. In some embodiments, heating can be provided by an electric radiator, an electric convector or other type of heating methods, while cooling is provided by compressor (i.e. by using the Carnot process), or by fresh water cooling or other cooling means.

The temperature adjustment system preferably generates as little pressure drop as possible, preferably it has sufficiently large emission surfaces so as to avoid unwanted condense water when cooling in warm and humid conditions, and is preferably able to maintain a cooling power that is stable over time and with minimal short term variations of supply air-temperature.

In preferred embodiments, heating/cooling is evenly distributed by means of heat pipes. Fins mounted on the heat pipes, with short distance to heat/cool source, can cover a wide cross section area of the air flow. Because the distance to the heat/cool source is short, efficient heat exchange can be achieved using relatively thin fins. In contrast, relatively thicker fins with lower thermal resistance are required using extruded heat sinks because of the longer distance to the heat source. Accordingly, the heat pipe system can effectively provide heat/cool transfer to a cross section area of air flow with comparably thinner fins resulting in lower air resistance and minimized pressure drop. Further, the short distance to the heat/cool source using heat pipes leads to an evenly distributed surface temperature which makes more efficient heat transfer per unit fin area. This leads to smaller temperature differences and thereby less risk of condense water accumulating on cooler areas of the fins.

It will be readily understood by one skilled in the art that a variety of different schemes for temperature adjustment may be employed. In systems that utilize a thermoelectric cooler (TEC), excess heat can be dissipated in variety of ways, including passive or active convection or active liquid cooling.

Preferred embodiments can stably maintain an air-temperature difference of supply air relative to ambient air at the level of the point of care with a minimal fluctuation. Fluctuation of the air-temperature difference is preferably kept within the range of the margin of measurement error, preferably ±0.1 1 °C. This stable air-temperature difference is preferably maintained at some point within the range of about 0.5 to <1 °C. In this manner, descending air-stream velocity can be "delicately balanced" between excessive velocity, which creates unwanted drafts, and sufficient velocity, which is just enough to break body convection currents.

The temperature control system (5) maintains a stable air-temperature difference between the descending supply air-stream enveloping the point of care (i.e. the breathing zone of a sleeping person) and the ambient air as measured at the level of the point of care. In one preferred embodiment, the temperature control system comprises two sensors and a control unit. One temperature sensor is placed in the supply air channel just after the temperature adjustment device (4). A second sensor is placed in such manner as to measure ambient air at the level of the personal breathing zone but outside the effective stream of supply air. The control unit is preferably programmed to collect data from the two sensors and to regulate voltage applied to the Peltier element so as to maintain a temperature difference within the optimal range. Sensors are preferably protected from any kind of radiation from surfaces so as to provide an accurate air-temperature measurement. Preferably, sensors have high sensitivity and minimal error margin, i.e. no more than ±0.05 °C.

The air supply nozzle (6) delivers a substantially laminar stream of supply air with minimal in-mixing of ambient air. In order that the velocity of the supply air-stream may be determined by difference in air-temperature from ambient air at the level of the point of care, supply air preferably exits the nozzle with a velocity that is just sufficient to overcome nozzle resistance. The initial dynamic pressure of supply air is rapidly diminished by static pressure of ambient air until a point is reached at which gravity alone (i.e. air-temperature difference) determines the rate of further descent. The nozzle preferably has minimal resistance whereby supply air may exit the nozzle with minimal dynamic pressure and, accordingly, whereby the point at which air-temperature difference alone determines the rate of further descent is reached well before the supply air-stream reaches the point of care. In some embodiments, the nozzle (6) can be replaced by or made in combination with one or more filters (2) as an integral part of the air supply nozzle or as the sole part delivering supply air.

A wide variety of nozzle shapes and sizes can be used. However, the rate at which initial velocity of supply air is diminished by static pressure of ambient air is affected by nozzle shape. Pitch length refers to the distance from the surface of the nozzle at which the cumulative effect of static pressure of ambient air counterbalances the dynamic pressure of supply air that has been set into flow with impulse just sufficient to overcome resistance in the nozzle. Preferably, a suitable nozzle has minimal pitch length. This permits gravity (i.e., air-temperature difference) to control the downward air flow velocity at a point well above the point of care. Short nozzle pitch length also ensures that supply air flow will introduce minimal disturbance of ambient air which in turn minimizes turbulences that arise when supply air meets still standing ambient air. In preferred embodiments, nozzle pitch length ends well before the point of care.

Preferably the pitch length, as defined by an air velocity of less than 0.2 m/s, should reach less than 20 cm from the air delivery device. In any case, the pitch length is preferably no longer than the distance between the air supply nozzle and the point of care. The prime factors determining the actual pitch length are shape of the nozzle and the composition the materials shaping the nozzle. A preferred nozzle is described in WO2005/017419. An air delivery nozzle with a substantially spherical shape as described is likely to cater for a larger effective operative area as compared to a flat air delivery nozzle, given identical air flow. However, both flat or spherical shaped nozzles can be used.

The substantially spherical shape has the advantage of being compact. Further the shape forces the air flow to be distributed over an increasing surface area. This reduces pitch length, in that the decrease in air velocity is dependent on friction between the supply air and ambient air. The spherical surface distributes supply air flow to a surface are that increases with approximately the square of the distance from the nozzle centre. The increasing surface area forces the velocity to decrease with approximately 1/(the square of the distance from the nozzle centre) giving the spherical nozzle a natural character with a short pitch length. In contrast, a flat delivery nozzle generates an air flow with a constant distribution area and a correspondingly longer pitch length.

Any alternative nozzle with similar characteristics of minimal pitch length and low disturbance of ambient air may be used.

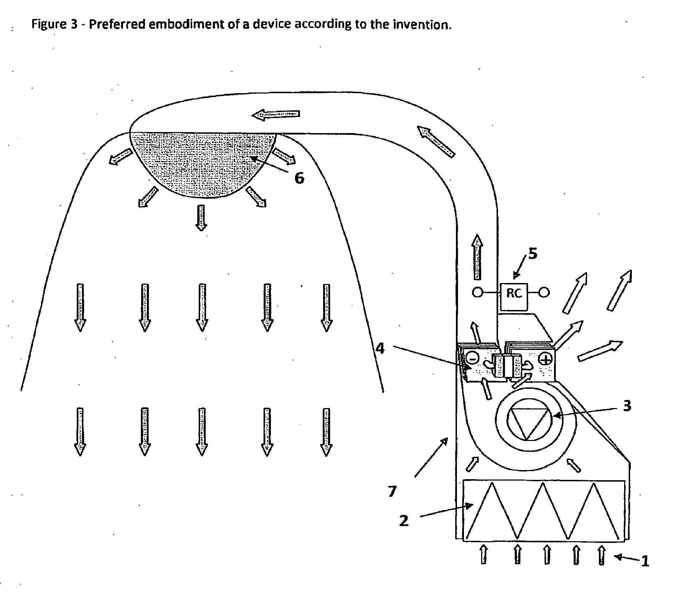

Figure 3 illustrates a preferred embodiment of a device for use in the invention. Ambient air (symbolized by shaded arrows, indicating flowing air) is taken in through the air inlet (1), which is situated at floor level at the bottom of the housing (7). Intake air is filtered by the filter (2), driven by action of the blower (3). An air-temperature adjustment device (4) is situated so as to provide both cooling and heating of the filtered supply air-stream. The device comprises a Peltier element with reversible voltage polarity connected via heat pipes to two sets of fins. One set of fins serves primarily to distribute cooling effect in the supply air-stream while the other set of fins serves primarily to provide dissipation of excess heat generated by the Peltier module. Parts of or the whole air-temperature adjustment device (4) may be situated before the filter (2) and/or the blower (3). Parts of or the whole air-temperature adjustment device (4) may also be situated in other parts of the device such as the nozzle (6). The temperature control device (5) comprises a control unit (square) and two sensors (circles). One sensor is placed in the supply air-stream while the other is placed in such manner so as to measure ambient air-temperature at the level of the personal breathing zone but outside the supply air-stream. The control unit, informed by air-temperature measurements from the sensors, regulates the temperature adjustment unit so as to maintain a stable air-temperature difference between the supply air and ambient air at the level of the point of care. Supply air is driven by action of the blower (3) out of the nozzle (6) with minimal impulse.

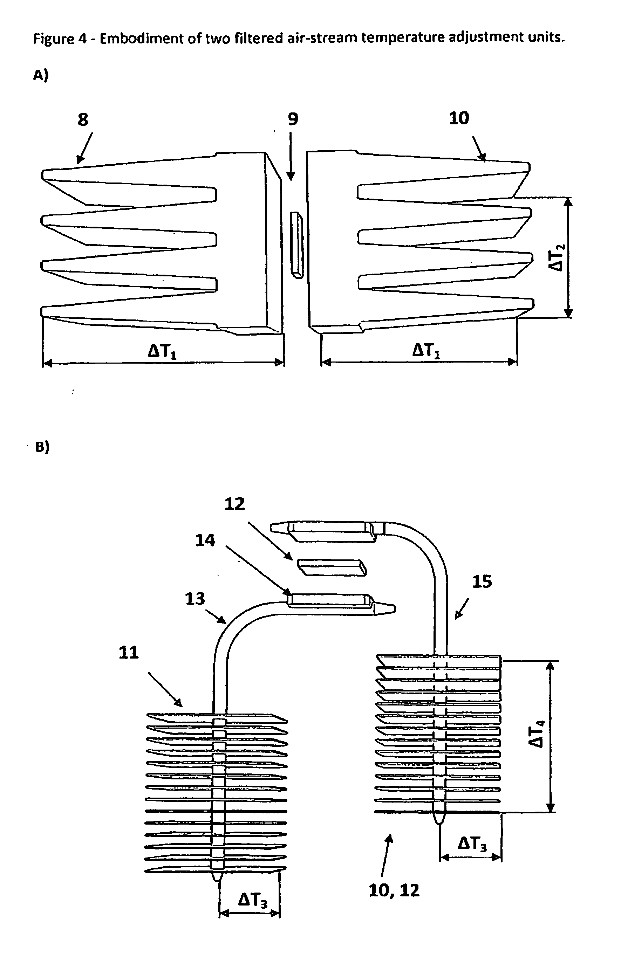

Figure 4 shows, in greater detail, an air-temperature adjustment unit (4) of a preferred embodiment. Figure 4A shows a TEC system with extruded heat sinks. In this system the TEC (9) distributes generated cooling effect on one side by interfacing an extruded heat sink (8). On the other side of the TEC heat is dissipated to a similar extruded heat sink (10). Figure 4B shows a heat pipe system. Here the TEC (12) interfaces a connection block (14) with at least the same area as the TEC. From here the cooling effect is transported to the fins by a heat pipe (13). At the warm side (15) the heat is transferred in the same way. The Peltier element is normally fitted with thermal grease or a thermal pad which increases the thermal conductivity of the thermal interface by compensating for the irregular surfaces of the components.

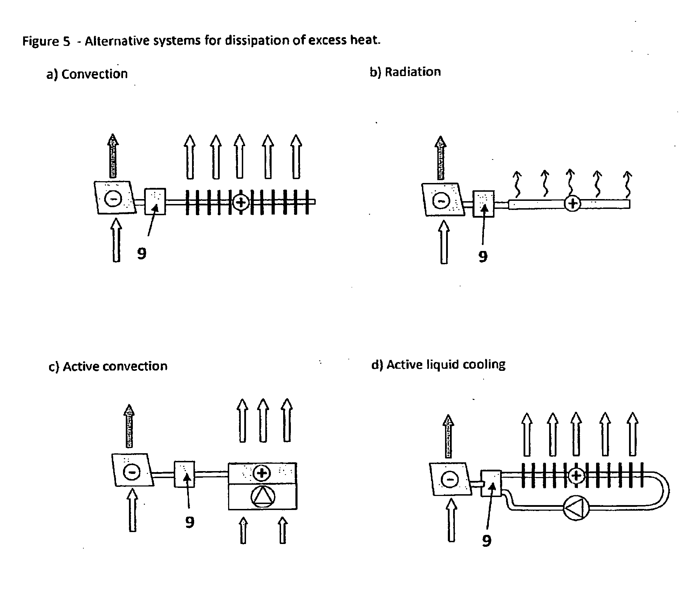

Figure 5 shows alternative systems for dissipating excess heat generated by the air-temperature adjustment unit. In a preferred embodiment using a TEC system, excess heat can be dissipated by convection, as shown in Figure 5a, by radiation, as shown in Figure 5b, by active convection, as shown in Figure 5c, or by active liquid cooling, as shown in Figure 5d. These alternative systems may act alone or in combination (i.e. by combining convection with radiation).

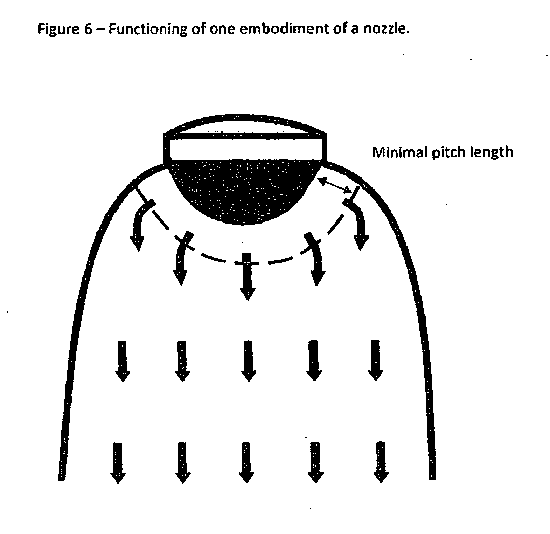

Figure 6 illustrates functioning of the nozzle (6) of the preferred embodiment shown in Figure 3. Shown is a schematic illustration of the functioning of the nozzle described in WO2005/017419.

Supply air is initially forced out of the nozzle with a slight velocity, about 0.2 m/s, just sufficient to overcome resistance in the nozzle. The spherical surface distributes supply air flow to a surface area that increases with approximately the square of the distance from the nozzle centre. Friction with ambient air dissipates the air flow velocity up to the pitch length, after which further descent of the supply air-stream is determined by air-temperature difference (gravity).

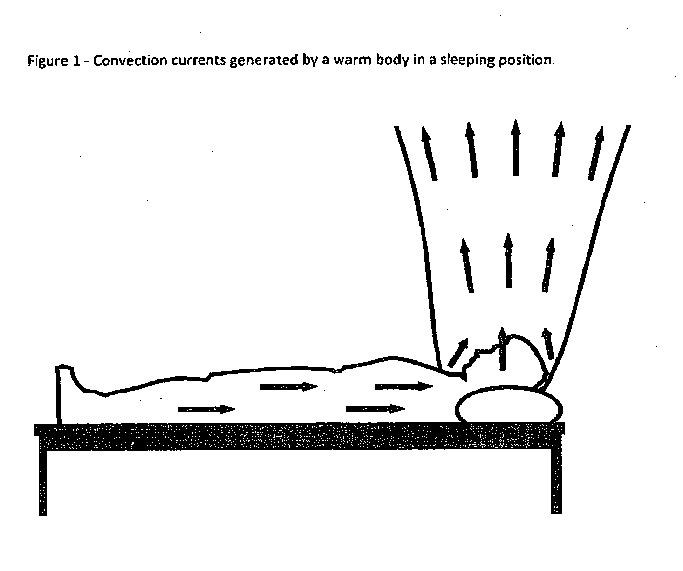

Figure 7 illustrates some alternative arrangements of preferred embodiments used in providing a controlled personal breathing zone. The air delivery nozzle, which can be spherical or flat or other shape, can be placed straight above the point of care, as shown in figures 7a and 7d. It can be slightly tilted and placed slightly off center of the point of care, as shown in figure 7b. It can be placed aside the point of care directing an impulse horizontally towards the point of care, as shown in figure 7c. In all settings gravity (temperature difference) defines a substantially downward directed air-stream (after initial forced impulse has been counteracted by friction with ambient air). The downward directed supply air-stream has sufficient velocity to displace conflicting body convection as illustrated in Figure 1. The preferred distance between the nozzle and the point of care is preferably within the range of about 20 cm to 80 cm.

In an unpublished multiple independent, double blind, randomized 52 week parallel clinical trial comparing active and placebo treatment with a TLA device, using one embodiment of the TLA device described in US 6,702,662, we have found that a relatively narrow range of conditions exists in which it is possible to reduce or remove the symptoms of asthma while avoiding drafts (caused by excessive velocity of the descending air-stream) while also avoiding the inability to displace warm body convection currents (caused by insufficient velocity of the treated air-stream) and at the same time minimizing in-mixing of ambient contaminated air.

It was surprisingly found that applying the treatment for 6 hours while the patient is sleeping provides for effective treatment of atopic dermatitis, hence there is no need for prolonged 24 hour treatment.

In preferred embodiments, a single filtered air-stream is subject to temperature adjustment and air-temperature of the filtered air can be carefully adjusted via a temperature control system to maintain, within the optimum range, an air-temperature difference between supply air and ambient air at the level of a personal breathing zone.

Reversible polarity of the TEC used to provide air-temperature adjustment permits the supply air-stream to be alternately cooled or heated, thereby providing necessary fine tuned control of descending air-stream velocity.

In one embodiment temperature controlled laminar air flow is used together with standard AD medicaments in the treatment of AD wherein the air is filtered and provided to a patient's personal breathing zone without in-mixing of contaminated ambient air said standard medicaments being inter alia topical corticosteroid and softening cream. Alternatively, treatment according to the above table reproduced from Walling and Swick (ref. 8) may be combined with TLA treatment. Optionally, said TLA treatment may be administered during sleep.

In another embodiment the temperature controlled laminar air flow is provided according to the preferred embodiment immediately above in such a fashion that body convection currents are braked without the induction of draught.

In yet another embodiment the TLA treatment according to the two embodiments immediately above is provided in such a fashion that the temperature of the air delivered into the treated air zone is 0.5 - <1 °C cooler than the ambient air surrounding the treated air zone. It is preferred to employ a temperature interval between 0.6-0.8 deg C.

In yet another embodiment TLA treatment is administered for at least 6 consecutive hours.

The preferred embodiments are exemplary only and not intended to limit the scope of the invention as defined by the claims.

The uses according to the claims of the invention may be taken to include the use of devices configured to provide the treatment according to e.g. claim 1.

### Example 1: Treatment of atopic dermatitis using a specific TLA device configuration

A warm human body lying in a bed causes a convection flow transporting a high concentration of allergens and airborne particles to the person's breathing zone. As shown in Figure 1, the warm body of a sleeping person generates such a convection air currents.

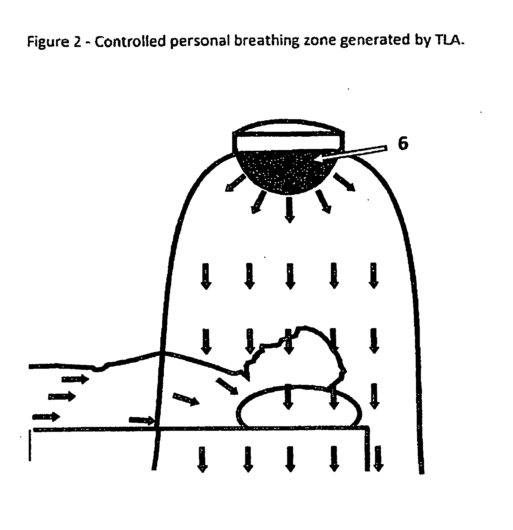

A TLA device such as the one illustrated in Figure 3 provides a descending stream of filtered air that has sufficient velocity to overcome these body convection currents, as shown in Figure 2. The air-temperature of the air delivered into the treated air zone is 0.5 to <1 °C (and preferably 0.6-0.8 °C) cooler than the ambient air surrounding the treated air zone resulting in the displacement of warm body convection currents without exposing the patient to an unpleasant draft. The zone of treated air provided by such devices may provide more than 95% reduction in airborne fine particle counts. The generation of such a controlled personal breathing zone that is substantially free of in-mixed, contaminated ambient air allows for reduction or removal of symptoms of atopic dermatitis without exposing the patient to an unpleasant draft.

### Example 2: Clinical study relating to treatment of atopic dermatitis

To compare the efficacy of a TLA device with a placebo device to reduce the degree of symptoms in patients with perennial allergic asthma and AD, sensitised to animal dander and/or house dust mites a clinical study was carried out.

The study was carried out as a multiple independent, double blind, randomized 52 week parallel trial comparing active and placebo treatment with the AP TLA device. For ethical reasons the randomization of patients was 2 to 1 for active and placebo treatment, respectively. Patients were randomized and included at visit 1 and a device was installed 2 to 4 weeks after inclusion.

The patient population consisted of male and female patients, 7 to 70 years of age, with established asthma and documented allergy to one or more of allergens, wherein a sub-population had been diagnosed with atopic dermatitis.

AP (Airsonett Protexo TLA device) active and placebo devices were used as test articles. In the placebo devices the filter was bypassed and the cooling turned off which prevented the air from reaching the breathing zone. The devices were installed by an independent person appointed by the Airsonett AB and sealed. No clinic personnel or trial monitor had access to the randomization list.

The trial was performed in accordance with the recommendations guiding physicians in biomedical research involving human Patients adopted by the 18th World Medical Assembly, Helsinki, Finland, 1964 and later revisions, ICH guidelines and good clinical practice.

Reports from a sub-population of patients diagnosed with atopic dermatitis indicated improvements in AD symptoms in this group of patients.

In case studies of 12 patients with moderate to severe AD, nocturnal TLA treatment resulted in significant improvements in the severity of atopic dermatitis, decreased itchiness, alleviated eczema eruptions, and in some cases reduction in treatment intensities with topical or systemic AD medication. Surprisingly, in spite of the treatment being limited to night time, it was effective in reducing AD symptoms in patients sensitized to wide range of aeroallergens (HDM, pet dander and pollen) Information in the form of written statements from patients and photos taken before and after TLA treatment from a total of 12 patients suffering from atopic dermatitis was evaluated and in all cases patients reported that they experienced a decrease in AD symptoms about 2-3 weeks into the trial, in some cases symptoms were completely removed.

Hence, it has surprisingly been found that nocturnal TLA treatment wherein a personal breathing zone is provided according to the present application can reduce or even remove symptoms of atopic dermatitis.

In the study the TLA device delivered air at a temperature 0.5 to <1°C cooler than the ambient air and at this setting there were no reports of patients experiencing drafts.

It was, furthermore, found that a temperature difference of 0.6 to 0.8 °C is particularly useful in minimizing draught while at the same time braking body convection currents. Moreover, the air temperature difference is selected such that the resulting air velocity is not high enough to result in the whirling up of particles present in e.g. bedding.

### Example 3

Eight children, aged 5 - 16, suffering from severe aeroallergen-induced AD had TLA treatment according to the present invention (cf. example 1) added to their medical treatment for at least 3 months (in certain cases up to four years). Patients were subjected to TLA treatment using a TLA device.

All patients were sensitized to house dust mites and/or pet allergens (allergy documented by skin prick test or equivalent), and had a history of treatment according to guidelines including topical moisturizers, potent glucocorticosteroids, immunomodulators, anti-itch products, antibiotics, one case cyclosporine, and different allergen avoidance measures. At base line all subjects suffered from persistent symptoms, itching, sleep disturbance and negative impact on Quality of Life despite medical treatment.

All eight children achieved a good improvement and 5/8 patients turned completely free from AD symptoms and could reduce their treatment to moisturizers only. 3/8 patients reduced treatment to moisturizers and intermittent use of mild topical glucocorticosteroids. Quality of life was significantly improved and none of the subjects reported to have any sleep disturbance. Symptom improvements and consequent medication reductions were achieved within one month in a majority of the cases. Short period without TLA treatment led to exacerbations of AD symptoms. Re-starting TLA treatment resulted in a quick improvement in severity of symptoms.

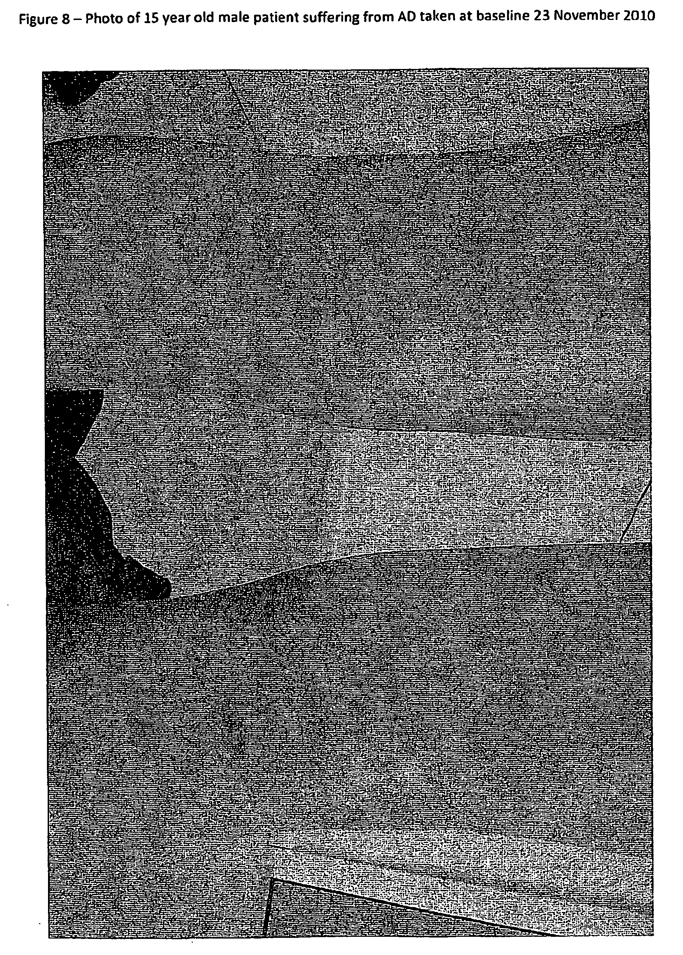

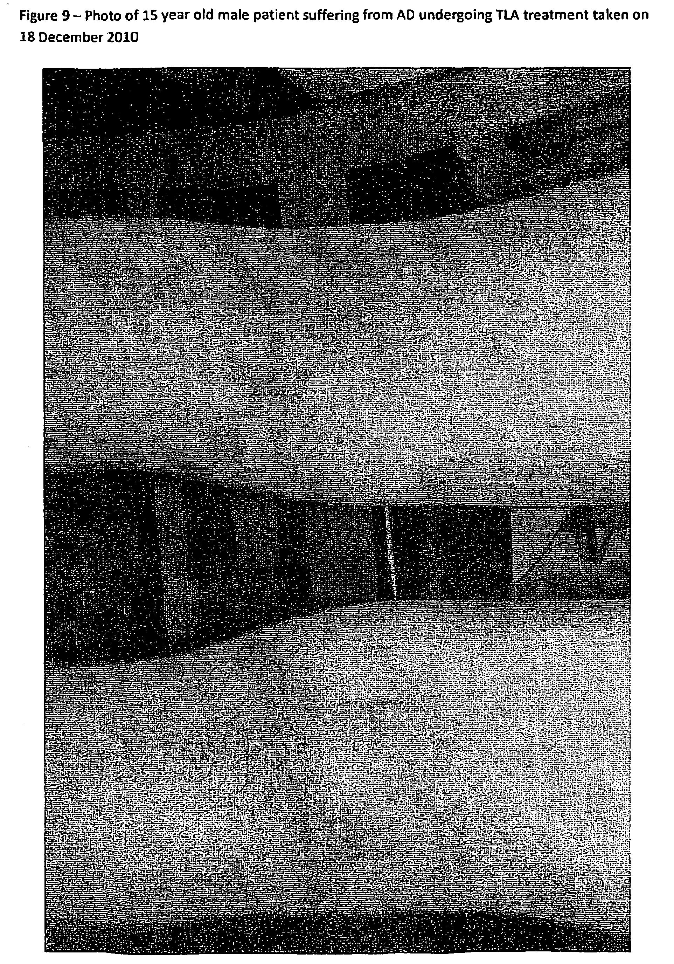

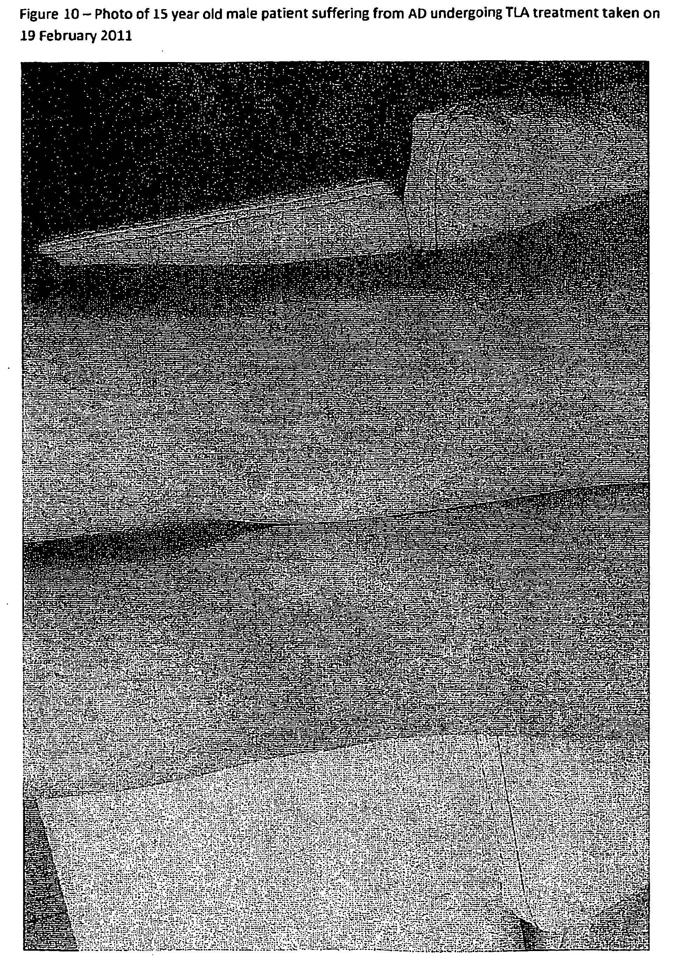

Figures 8-10 show the reduction of symptoms in one of the above mentioned patients, specifically a 15 year old male suffering from AD. Figure 8 is taken at base line before initiation of TLA treatment (23 November 2010), figure 9 shows a significant decrease in AD symptoms after about one month (18 December 2010) and figure 10 shows almost no symptoms after about three months of TLA treatment (19 February 2011).

### Example 4

A 49 year old woman with severe bleeding eczema, comorbid asthma and frequent awakening during night from itching and asthma was undergoing treatment for several years with cutaneous corticosteroid and softening cream (5-8 times/day including night time) said treatment having an unsatisfactory effect.

After 2 months of nightly TLA treatment according to the present invention (cf. example 1) the eczema improved significantly and the itching and bleeding stopped. The patient was able to sleep throughout the night without awakening. Moreover, the patient became more alert and managed to walk long distances after treatment according to the present invention which was not possible before TLA treatment. Corticosteroid treatment of eczema and reduced asthma treatment was stopped and bronchial inflammation as measured by Fractional Exhaled Nitric Oxide (FENO) was reduced from 36 to 17ppb (normal level <25 ppb). The positive effect of TLA treatment was sustained over time with continuous treatment.

### References

1. Mancini AJ, Kaulback K, Camlin SL. The socioeconomic impact of atopic dermatitis in the United States: a systematic review. Pediatr Dermatol. 2008;25(1):1-6.
2. Wahn U, Warner J, Simons FE, et al. EPAAC Study Group. IgE antibody responses in young children with atopic dermatitis. Pediatr Allergy Immunol. 2008;19(4):332-336.
3. Hon KL, Leung TF, Lam MC, et al. Which aeroallergens are associated with eczema severity? Clin Exp Dermatol. 2007;32(4):401-404.
4. Holm L, van Hage-Hamsten M, Ohman S, Scheynius A. Sensitization to allergens of house-dust mite in adults with atopic dermatitis in a cold temperature region. Allergy. 1999;54(7):708-715.
5. Schafer T, Heinrich J, Wjst M, et al. Association between severity of atopic eczema and degree of sensitization to aeroallergens in schoolchildren. J Allergy Clin Immunol. 1999; 104(6):1280-1284.
6. Sanda T, Yasue T, Oohashi M, Yasue A. Effectiveness of house dust-mite allergen avoidance through clean room therapy in patients with atopic dermatitis. J Allergy Clin Immunol. 1992;89(3):653-657.
7. Hostetler SG, Kaffenberger B, Hostetler T, Zirwas MJ. The role of airborne proteins in atopic dermatitis. J Clin Aesthet Dermatol. 2010 Jan;3(1):22-31.
8. Walling, H & Swick, B.L. Update on the management of chronic eczema: new approaches and emerging treatment and emerging treatment options. Clin Cosm and Investing Dermat. 2010:3 99-117.

## Claims

1. Temperature controlled laminar air flow for use in the treatment of atopic dermatitis wherein the air is filtered and provided to a patient's personal breathing zone without in-mixing of contaminated ambient air.

2. The temperature controlled laminar air flow for use according to claim 1 wherein body convection currents are braked without the induction of draught.

3. The temperature controlled laminar air flow for use according to any of claims 1-2 wherein the treatment is administered during sleep.

4. The temperature controlled laminar air flow for use according to any of the claims 1-3 wherein treatment is administered for at least 6 consecutive hours.

5. The temperature controlled laminar air flow for use according to any of the claims 1-4 wherein the temperature of the air delivered into the treated air zone is 0.5 - <1 °C cooler than the ambient air surrounding the treated air zone.

6. The temperature controlled laminar air flow for use according to any of the claims 1-5 wherein the temperature of the air delivered into the treated air zone is 0.6 - 0.8 °C cooler than the ambient air surrounding the treated air zone.

## Patentansprüche

1. Temperaturgesteuerter laminarer Luftstrom zur Anwendung in der Behandlung von atopischer Dermatitis, wobei die Luft filtriert wird und in die persönliche Atmungszone eines Patienten zugeführt wird ohne Einmischung kontaminierter Umgebungsluft.

2. Temperaturgesteuerter laminarer Luftstrom zur Anwendung nach Anspruch 1, wobei Strömungen von Körperkonvektion abgebremst werden ohne Zugluft herbeizuführen.

3. Temperaturgesteuerter laminarer Luftstrom zur Anwendung nach einem der Ansprüche 1-2, wobei die Behandlung während Schlaf eingesetzt ist.

4. Temperaturgesteuerter laminarer Luftstrom zur Anwendung nach einem der Ansprüche 1-3, wobei die Behandlung für mindestens 6 aufeinanderfolgende Stunden eingesetzt ist.

5. Temperaturgesteuerter laminarer Luftstrom zur Anwendung nach einem der Ansprüche 1-4, wobei die temperatur der Luft, die in die Zone von behandelter Luft zugeführt wird, 0.5 - <1 ºC kälter ist als die Umgebungsluft, die die Zone von behandelter Luft umgibt.

6. Temperaturgesteuerter laminarer Luftstrom zur Anwendung nach einem der Ansprüche 1-5, wobei die temperatur der Luft, die in die Zone von behandelter Luft zugeführt wird, 0.6 - 0.8 ºC kälter ist als die Umgebungsluft, die die Zone von behandelter Luft umgibt.

## Revendications

1. Flux d'air laminaire thermocommandé pour utilisation dans le traitement de la dermatite atopique, dans lequel l'air est filtré et fourni à la zone de respiration personnelle du patient sans y mélanger de l'air ambiant contaminé.

2. Flux d'air laminaire thermocommandé pour utilisation selon la revendication 1, dans lequel des courants de convections du corps sont freinés sans induction de courant d'air.

3. Flux d'air laminaire thermocommandé pour utilisation selon l'une quelconque des revendications 1 à 2, dans lequel le traitement est administré pendant le sommeil.

4. Flux d'air laminaire thermocommandé pour utilisation selon l'une quelconque des revendications 1 à 3, dans lequel le traitement est administré pendant au moins 6 heures consécutives.

5. Flux d'air laminaire thermocommandé pour utilisation selon l'une quelconque des revendications 1 à 4, dans lequel la température de l'air fournie dans la zone d'air traitée est 0,5 - <1 °C plus froid que l'air ambiant qui entoure la zone d'air traitée.

6. Flux d'air laminaire thermocommandé pour utilisation selon l'une quelconque des revendications 1 à 5, dans lequel la température de l'air fournie dans la zone d'air traitée est 0,6 - 0,8°C plus froid que l'air ambiant qui entoure la zone d'air traitée.
